# EUROPEAN PATENT APPLICATION

(11) **EP 3 824 845 A1**
(43) Date of publication of application: **26.05.2021**
(21) Application number: 20275172.3
(22) Date of filing: 19.11.2020
(51) Int. Cl.: A61F 2/07, A61F 2/95

(54) **STENT GRAFT TRIGGER WITH INDIRECT FIXATION**

(30) Priority: 22.11.2019 US 201962939219 P
(71) Applicant: Cook Medical Technologies LLC, Bloomington, IN 47404 (US)
(72) Inventor: SPINDLER, Ralf, Solsberry, IN Indiana 47459 (US); SUMANASINGHE, Ruwan, Carmel, IN Indiana 46032 (US)
(74) Representative: Williams Powell

(57) **Abstract**

Disclosed herein are systems and methods for delivering a medical device to a body vessel of a patient. The medical device may be a stent graft (230), and the delivery assembly may include at least one loop that connects an end stent of the stent graft to a trigger wire (240) of the delivery assembly, thereby providing indirect connection between the device to be delivered and the trigger wire (240).

## Description

### FIELD

The present disclosure relates to an apparatus and method for delivering an implantable medical device, particularly a stent graft.

### BACKGROUND

The statements in this section merely provide background information related to the present disclosure and may not constitute prior art.

In aortic intervention, stented endovascular grafts are currently used for the treatment of aneurysms and aortic dissections. Stent grafts are commonly placed into such diseased vessels for structural support and to exclude blood flow to aneurysms. Therefore, they may be able to reduce or prevent further vessel expansion, and thus can reduce the likelihood of aneurysm rupture.

Proper placement of stent grafts aids in the success of such treatments. Good wall apposition and anchoring can help to reduce the occurrence of Type I and Type III endoleaks.

Different release mechanisms are currently used in endovascular graft delivery devices to unfold and/or attach stent grafts to vessel walls. These stents may include top stents that are located at an end of the endovascular graft, distal stents, and other stents that are located within the main body of the endovascular graft. As most stents used in these devices have barbs that attach to the vessel, the slow or controlled release of these stents or stented grafts can allow for accurate placement of the graft. If these stents are released without a controlled release mechanism during unsheathing, immediate engagement of the barbs is possible, which in turn may prevent readjustment of the positioning of the endograft.

Many such release mechanisms employ wires (such as nickel/titanium or stainless steel wires) to activate the release of the stent or stent graft. This release mechanism is designed such that it is easy to use, that it has a low profile, and that it functions reliably.

It has been a challenge to develop a medical device delivery assembly that quickly and reliably releases a stent graft or a stent thereof in such a way that the graft is repositionable.

### SUMMARY

Aspects of the present invention seek to provide an improved system, method or medical device assembly.

According to an aspect of the invention, there is provided a system as in claim 1.

According to an aspect of the invention there is provided a medical device assembly as in claim 15.

According to one aspect of the present disclosure, a system for deploying at least a portion of a stent is described. The system may include a cannula comprising a lumen. The system may include a guide member disposed over the cannula. The guide member may include an outer surface, and the guide member may define at least one curved channel therein. The at least one curved channel may define a trigger wire lumen. The guide member may include at least one access port through the outer surface to the curved channel. The system may include a trigger wire disposed in the trigger wire lumen.

According to another aspect of the present disclosure, a medical device assembly is described. The medical device assembly may include a cannula comprising a lumen, which may be a wire guide lumen. The medical device assembly may include a guide member disposed over the cannula. The guide member may have an outer surface and may comprise at least one curved channel therein. The at least one curved channel may define a trigger wire lumen which may be distinct from, or not in fluid communication with, the wire guide lumen. The guide member may include at least one access port through the outer surface to the curved channel. The medical device assembly may include a trigger wire disposed in the trigger wire lumen defined by the curved channel. The medical device assembly may include at least one loop disposed about the trigger wire and extending through the access port. The medical device assembly may include a stent graft disposed about the cannula, the stent graft including an end stent having an apex. The apex of the end stent may extend through the loop.

According to another aspect of the present disclosure, a method of delivering a stent graft is provided. The method may include moving a trigger wire distally in a medical device assembly that includes a trigger wire surrounded by a loop, the loop restraining an apex of an end stent of the stent graft. Moving the trigger wire distally such that the trigger wire passes completely through the loop may cause the apex to no longer be restrained. The method may include, when the trigger wire is moved distally, a plurality of apices being sequentially released. The method may include, when the trigger wire is moved distally, a plurality of apices being simultaneously released.

Further areas of applicability will become apparent from the description provided herein. It should be understood that the description and specific examples are intended for purposes of illustration only and are not intended to limit the scope of the present disclosure.

### DRAWINGS

In order that the disclosure may be well understood, there will now be described various forms thereof, given by way of example, reference being made to the accompanying drawings, in which:
FIG. 1A is a view of an apex of an end stent of a stent graft being in direct connection with a trigger wire of a delivery assembly;
FIG. 1B is a view of an apex of an end stent of a stent graft connected to a trigger wire of a delivery assembly by a loop;
FIG. 2 is a perspective view of an end portion of a medical device delivery assembly engaged with the end stent of a stent graft;
FIG. 3 is a perspective view of an end portion of a medical device delivery assembly including a sleeve and engaged with the end stent of a stent graft;
FIG. 4A is cross-sectional view of the delivery assembly of FIG. 2 taken across line 4;
FIG. 4B is cross-sectional view of another example of a delivery assembly in accordance with the present disclosure;
FIG. 4C is a cross-sectional view of another example of a delivery assembly in accordance with the present disclosure;
FIG. 5 is a close-up perspective view of the loops of a medical device delivery assembly according to one example of the present disclosure;
FIG. 6 is a close-up perspective view of the loops of a medical device delivery assembly according to another example of the present disclosure;
FIG. 7A is a perspective view of a guide member constructed in accordance with the principles of the present disclosure;
FIG. 7B is a view of the guide member of FIG. 7A illustrating additional features of said guide member;
FIG. 8A is a view of a different form of a guide member for use in a medical device delivery assembly constructed in accordance with the principles of the present disclosure;
FIG. 8B is a cross-sectional view of the guide member of FIG. 8A taken along line 7B;
FIG. 9 is a perspective view of a medical device delivery assembly including a stent graft mounted thereon;
FIG. 10 is a perspective view of an end stent of a stent graft engaged with loops of a medical device delivery assembly in accordance with one example of the present disclosure;
FIG. 11 is a perspective view of an end stent of a stent graft engaged with loops of a medical device delivery assembly in accordance with another example of the present disclosure; and
FIG. 12 is a perspective view of an end stent of a stent graft engaged with loops of a medical device delivery assembly in accordance with another example of the present disclosure.

The drawings described herein are for illustration purposes only and are not intended to limit the scope of the present disclosure in any way.

### DETAILED DESCRIPTION

The following description is merely exemplary in nature and is not intended to limit the present disclosure, application, or uses. It should be understood that throughout the drawings, corresponding reference numerals indicate like or corresponding parts and features.

In the present application, the term "proximal end" is used when referring to that end of a medical device closest to the heart after placement in the human body of the patient, and may also be referred to as the inflow end (the end that receives fluid first), and the term "distal end" is used when referring to that end opposite the proximal end, or the one farther from the heart after its placement, and may also be referred to as the outflow end (that end from which fluid exits).

Although numerous examples of trigger wire/release systems for the delivery of stent grafts exist and have been commercialized, such systems generally involve the direct connection of the device to be delivered to the delivery system. A release mechanism that secures a portion of the device indirectly to the delivery system may provide increased freedom of motion to the portion of the device to which it is connected. In some examples, the portion of the device that is connected may be a top stent; that is, the most proximal stent of the stent graft, which in some cases may be a suprarenal stent. However, other portions of the device, including other stents, may also be connected to the delivery system. Such indirect connection may also provide for a simplified release mechanism, including reducing the total number of trigger wires used in the delivery assembly.

An assembly providing for indirect connection between the device to be delivered and the assembly itself may provide for better control of release of the device in the body of the patient, and may also allow for greater freedom of movement of the device during delivery. Freedom of transverse, axial and rotational movement within the confines of the vessel walls can allow the device to conform itself to the natural vessel angulations, which in turn can improve wall apposition and sealing at the downstream stent sealing zone, and can reduce uneven localized forces experienced on barbs and at attachment sites, thereby reducing the changes of type I and type III endoleaks, perhaps even preventing them altogether.

As used herein, the term "direct" when used in reference to a connection between a stent portion of a stent graft to be released by a trigger wire and the trigger wire of the stent graft delivery assembly itself means that the trigger wire contacts the stent portion of the stent graft without contacting an intermediate component. "Indirect" means that the contact between the trigger wire and the stent portion of the stent graft to be released by the trigger wire is bridged by at least one additional component that is not the trigger wire and is not the stent portion of the stent graft.

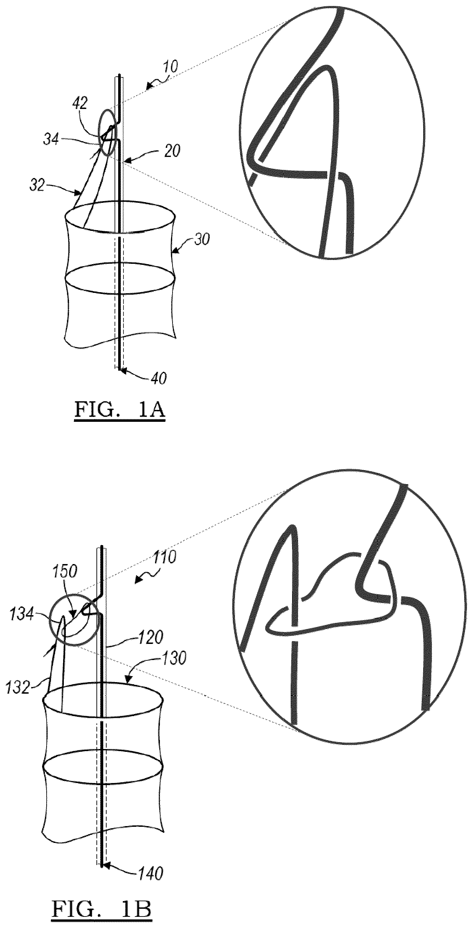

FIG. 1A illustrates an example of a delivery assembly 10 having a direct connection between the device to be delivered and a trigger wire. In FIG. 1A, a stent graft 30 is disposed about cannula 20 and an apex 34 of top stent 32 (or end stent) of the stent graft 30 is directly connected to a trigger wire 40 of the delivery assembly 10 at catch 42. This connectivity can be observed in a number of commercially-available stent graft release systems. In this example, the cannula 20 has a lumen in which the trigger wire or trigger wires are contained, without sharing the space with a guide wire if one is employed.

FIG. 1B illustrates a delivery assembly 110 in which stent graft 130 is indirectly connected to trigger wire 140. In FIG. 1B, the top stent 132 of stent graft 130 is indirectly connected near apex 134, via loop 150, to trigger wire 140. The top stent 132 may have a sinuous structure that results in a plurality of apices 134. The trigger wire 140 runs through cannula 120 of the delivery assembly 110 and can be manipulated by a medical practitioner to release the stent graft 130 when it is at the desired location in the vasculature of the patient. Such an arrangement may provide the benefit of a greater degree of motion of the top stent 132 during deployment, providing better adjustability of the diameter of the stent graft 130, and providing the delivery assembly 110 with a relatively low profile.

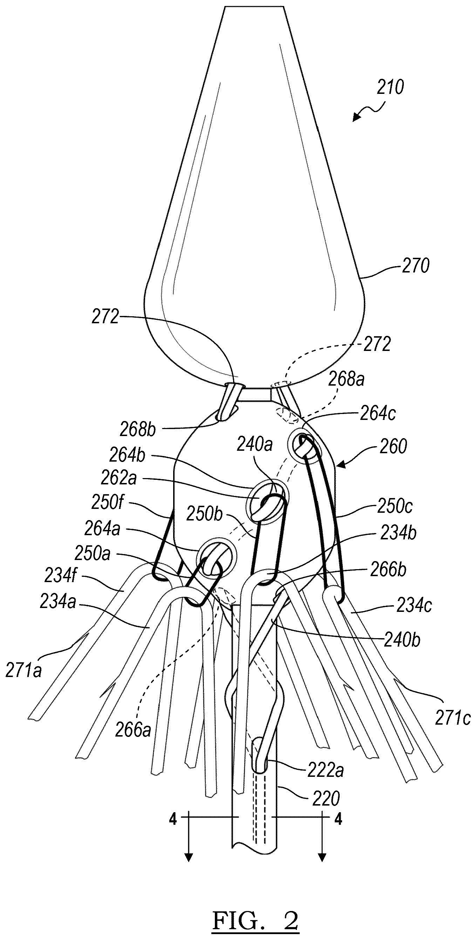

FIG. 2 illustrates one example of a portion of a delivery assembly 210 constructed in accordance with the principles of the present disclosure. The view of FIG. 2 illustrates a proximal portion of the assembly 210, with tip 270 being the most proximal portion of the assembly 210. Tip 270 may be made of a nylon or nylon copolymer for increased flexibility. The delivery assembly 210 has a stent graft 230 disposed thereon, but for clarity of illustration, only portions of top stent 232 (including apices 234a, 234b, 234c, and so forth) are shown.

In the delivery assembly 210 as shown in FIG. 2, cannula 220 may be connected to guide member 260. The cannula 220 may comprise two trigger wire lumens therethrough, through which the two trigger wires 240a and 240b extend. That is, the cannula 220 may instead include a central guide wire lumen, and may include separate trigger wire lumens formed therethrough. There may be individual lumens for each trigger wire, or the trigger wire lumens may be shared by multiple trigger wires.

The trigger wires 240a/240b may extend out of the cannula 220 via openings 222a/222b, respectively (222b being on the opposite face of the cannula 220 as illustrated, not shown). The trigger wire 240a then enters an entry port 266a in the guide member 260 and extends proximally and in a helical fashion through a channel 262a (which itself is curved, or helical) in the interior of guide member 260, as will be described later in this disclosure. The channel 262a may have a series of openings (264a/264b/264c in the variation as illustrated in FIG. 2) that provide the channel 262a with fluid communication to the external environment. This also allows for access to portions of the trigger wire 240a. The edges of the openings 264 may be chamfered, as shown. The trigger wire 240a may then extend proximally through exit port 268a into an aperture 272 of the tip 270, where an end of the trigger wire 240a or 240b may be at least temporarily fixed or captured, or in some cases may remain unfixed. The path of trigger wire 240b on the opposite side of delivery assembly 210, not shown, may have similar features to that of the path of trigger wire 240a. The trigger wires 240a/240b may have substantially straight proximal ends while engaged, or may have a looped configuration. The overall effect of such a construction may be to reduce the number of trigger wires employed by the delivery assembly.

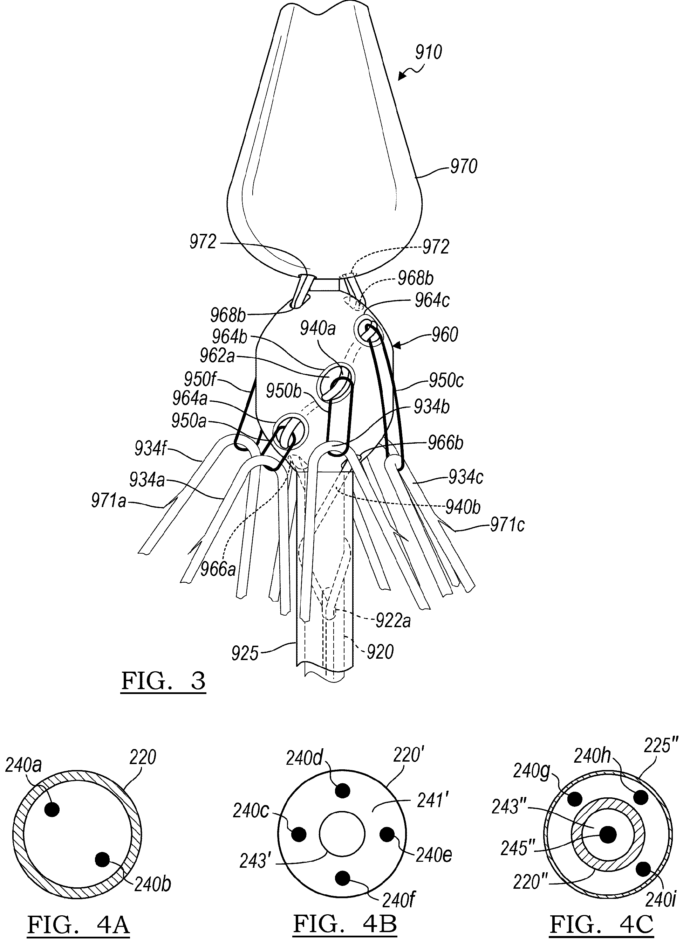

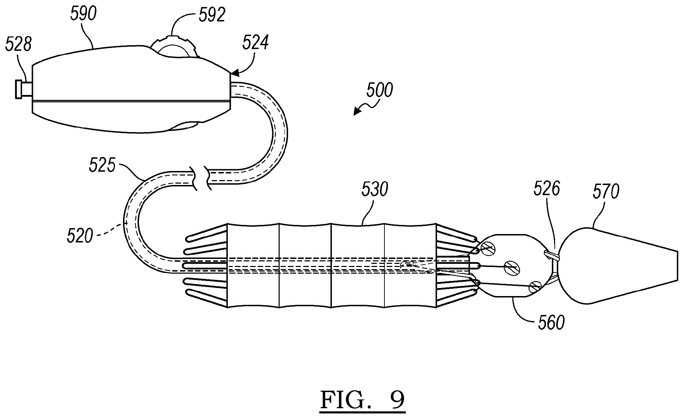

In the illustrated delivery assembly 210 of FIG. 2, the guide member 260 has six openings, including openings 264a/264b/264c, through its outer surface. The openings open into channel 262a. Likewise, openings 264d/264e/264f open into channel 262b (not visible in the illustration). The trigger wire 240a is accessible through openings 264a/264b/264c, and the trigger wire 240b is accessible through openings 264d/264e/264f. The two trigger wires 240a and 240b may run through trigger wire lumens defined in the cannula 220 of delivery assembly 210, as seen in FIG. 4A, and may be operably connected to a handle (described with reference to FIG. 9). The trigger wires 240a/240b may be made of any suitable biocompatible or hemocompatible wire, including but not limited to a nickel/titanium alloy or stainless steel.

In another example, the delivery assembly may have a cross-section as illustrated in FIG. 4B. In this example, a guide wire lumen 243' runs centrally through the body 241' of the cannula 220', with individual trigger wire lumens 240c/240d/240e/240f running through the body 241'. This allows the delivery assembly to be introduced over a guide wire. The cannula in this example is of sufficiently solid construction to endure forces experienced by the assembly, while being flexible enough to be adjustable in the anatomy.

FIG. 4C illustrates another example of a delivery assembly cross section. In this example, cannula 220" is surrounded by sleeve 225", which contains trigger wires 240g/240h/240i. The cannula 220" defines a lumen 243" in which a guide wire 245" can be contained.

In the illustrated example, the top stent 232 of stent graft 230 has six apices 234a-234f, and the two trigger wires 240a/240b of delivery assembly 210 each pass by three openings (256a-256c and 256d-256f) in the guide member 260, providing six total openings, one for each apex of the top stent 232. In some examples, the delivery assembly 210 may be constructed to have a number of openings equal to the number of apices of the top stent of the endograft to be delivered by the delivery assembly. Those of skill in the art will appreciate that designs having differing numbers of trigger wires, openings in the guide member, and channels formed in the guide member can be constructed in accordance with the principles of the present disclosure, depending on the specific application for which the delivery assembly is intended.

The guide member 260 may be of solid construction, aside from the channels 262a and 262b, or may have hollow portions other than the channels 262a/262b. As illustrated, the guide member 260 has a substantially ellipsoid shape, but other shapes may be suitable as well, including spherical, rounded prismatic, and so forth. The channels 262a/262b may be machined in a solid piece of precursor material, or it may be additively manufactured to precisely control the size and shape of the channels for the trigger wires. The guide member 260 may be constructed without sharp edges at either end such that it will not damage the graft or the vessel during operation.

The guide member 260 may be made of any suitable material. In one example, the guide member 260 may be made of a metal, including but not limited to stainless steel, titanium, and other metals or metal alloys with biocompatible and hemocompatible properties. In another example, polymers including polyether ether ketone (PEEK), polyoxymethylene (DELRIN), polyethylene, and other biocompatible and hemocompatible polymers with comparable properties may be used.

As shown in FIG. 2, loops 250, such as illustrated loops 250a/250b/250c/250f (along with loops 250d and 250e, not shown) provide a connection between the stent graft 230 and the delivery assembly 210. In this way, the device to be delivered is indirectly connected to the delivery assembly. The loops 250a-250f are not considered to be a portion of the stent graft 230, and may instead be considered to be a component of the delivery assembly 210 in some aspects, even when the loops are left with the stent graft inside the vasculature following delivery. Despite not being considered a portion of the stent graft 230, the loops 250a-250f may nonetheless be delivered with the stent graft 230 to the body lumen of the patient. The loops 250 interact minimally with blood flow.

The loops 250, when engaged with the stent apices 234 of the top stent 232 of the stent graft 230 and the trigger wire 240 of the delivery assembly 210, define an attached state (or delivery state) for the stent graft 230. This attached state allows freedom of motion to the stent apex such that it can move transversely, axially, and partially rotate about the cannula 220 so that this motion may enable the main body of the stent graft 230 (especially the proximal sealing section) to conform to the vessel wall during unsheathing of the graft prior to stent release by the trigger wires 240a/240b. This is in contrast to known assemblies, particularly abdominal aortic aneurysm (AAA) stent grafts having a suprarenal stent, wherein the orientation of the delivery assembly in the vessel plays a larger role in governing the orientation of the remainder of the graft in the vessel, which in some cases may reduce efficiency in sealing and less perfect vessel wall apposition upon deployment.

The loops 250 may be made from any suitable biocompatible material. For instance, they may be made of a nondegradable suture material, including but not limited to polypropylene, polyester, Dyneema, or another hemocompatible pure or blended material. The loops 250 may also be made of a biocompatible or hemocompatible wire, such as one made from a nickel/titanium alloy or stainless steel.

An end portion of another delivery assembly 910 is illustrated in FIG. 3. In FIG. 3, like components are labeled similarly to those that are illustrated in FIG. 2. Delivery assembly 910 additionally includes sleeve 925, which passes over and surrounds cannula 920 and covers a length of trigger wires 940a and 940b, thereby securing them and decreasing the profile of the delivery assembly 910.

The sleeve 925 may be made of a biocompatible polymer, and may fit tightly enough to restrain the trigger wires 940a/940b in the radial dimension, but loosely enough that it does not interfere with movement in the longitudinal dimension when the trigger wires 940a/940b are manipulated. In this way, the trigger wires 940a/940b may contact, or run along the length of, the cannula 920 when the sleeve 925 is employed. The sleeve 925 may extend over opening 922a and may extend over portions of the trigger wires 940a/940b. In some examples, the sleeve 925 may have a hole through it for positioning over or substantially over the opening 922a, to allow the trigger wires 940a/940b to pass through.

As illustrated in FIG. 3, the sleeve 925 may contact, or terminate just distal of, the guide member 960. In some forms, the sleeve 925 may extend further in the proximal dimension, covering at least a portion of the guide member 960. In another form, the sleeve 925 may extend over the entire guide member 960, but may still allow for the release of loops 950 by the trigger wires 940a/940b. In another form, the sleeve 925 may cover a portion of the tip 970.

The guide member 960 may be attached or fixed to the cannula 920, which may be made of a metal; to the sleeve 925 which may be made of a plastic or a polymer; or to both the cannula 920 or the sleeve 925.

The various delivery assemblies illustrated and described throughout this explanation may optionally include a sleeve similar to sleeve 925. For clarity, the sleeve has been omitted in figures other than FIG. 3.

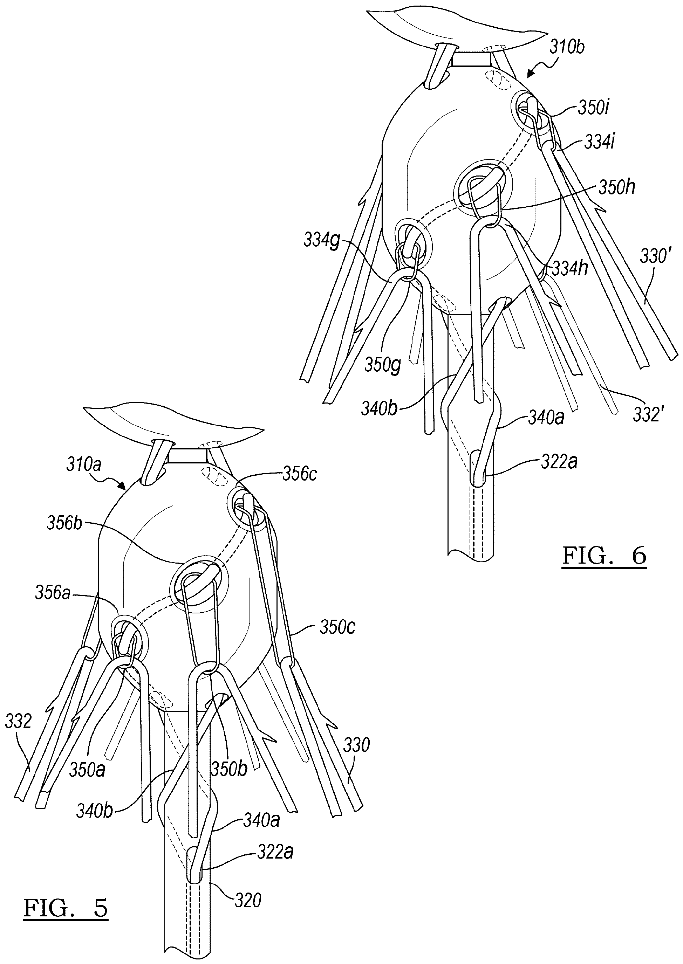

A number of variations on loops may be useful for indirect connection of the stent graft to the delivery assembly according to the principles of the present disclosure. In one example, and as shown in FIG. 5, the loops 350a/350b/350c of delivery assembly 310a may include loops of varying sizes depending on the distance from an opening 356a/356b/356c to the respective apex of the top stent 332 to which it is to provide indirect connection. As shown, loop 350c has a greater length than loop 350b, and loop 350b has a greater length than loop 350a. Such a configuration may be useful when the top stent 332 of the stent graft 330 has apices at the same or at similar heights compared to the remainder of the stent graft 330. In some cases, multiple loops may share a single opening.

In a variation shown in FIG. 6, by contrast, the delivery assembly 310b as configured has the same sized loops 350g/350h/350i regardless of position. Such a construction may be of use when the top stent 332 of the stent graft 330 has apices (such as 334g/334h/334i) of varying height.

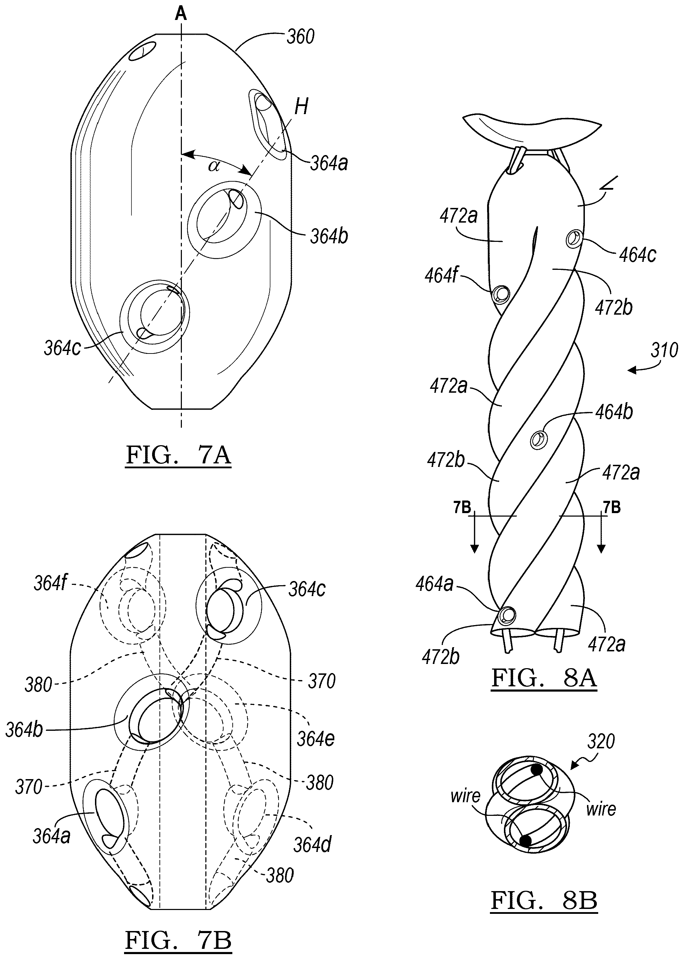

FIG. 7A illustrates the helical character of a curved channel 362 of a guide member 360 constructed in accordance with the principles of the present disclosure. The longitudinal axis A of the delivery assembly 310 is shown, as is a line H which is taken through a portion of the channel 362. The lines A and H intersect to define helical angle α. Typically, the measure of angle α will be between about 5 degrees to about 85 degrees, or about 10 degrees and about 75 degrees, or between about 15 degrees and about 60 degrees, or about 20 degrees and about 45 degrees, or about 25 degrees and about 40 degrees, or about 30 degrees and 35 degrees, or about 30 degrees, or about 35 degrees, or about 45 degrees, or about 60 degrees. FIG. 7B illustrates the inner workings of guide member 360 of FIG. 7A. Channel 362a curves along the length of the guide member 360, and channel 362b mirrors it (that is, one of the channels may be a left-handed helix and the other may be a right-handed helix), formed at the same helical angle as channel 362a.

FIG. 8A shows a portion of a delivery assembly 410 which has a guide member 460 formed in a different shape. The guide member 460 includes two tubes 472a and 472b defining the trigger wire channels, tubes 472a and 472b being twisted about one another to define the guide member 460. Similar to other guide members illustrated herein, the guide member 460 has a number of openings 464a/464b/464c/464f shown for access to the trigger wire housed therein from the outside of the assembly 410. FIG. 8B shows a cross-sectional view of the guide member 460 taken across line 7B in FIG. 8A.

A full view of an exemplary delivery system 500 is shown in FIG. 9 (not to scale). The delivery assembly 510 has a stent graft 530 mounted over the cannula 520, which extends from handle 590, through guide member 560, and the proximal end 526 connects to tip 570. Sleeve 525 is disposed over cannula 520. The distal end 524 of the cannula 520 is connected to the handle 590. An access port 528 may be in fluid communication with the cannula 520. In some examples, the handle 590 may include a thumbwheel 592, which can be operably connected to the trigger wires 540a/540b. Rotation of the thumbwheel in a first rotational direction may cause one or both of the trigger wires 540a/540b to move distally, thereby releasing the stent graft 530. Loops 550 connect the top stent 532 of the stent graft 530 indirectly to the delivery assembly 510.

In some examples, the stent graft may be crimped over the delivery assembly, and/or may be held in place by a sleeve positioned over the system including the stent graft and the delivery assembly.

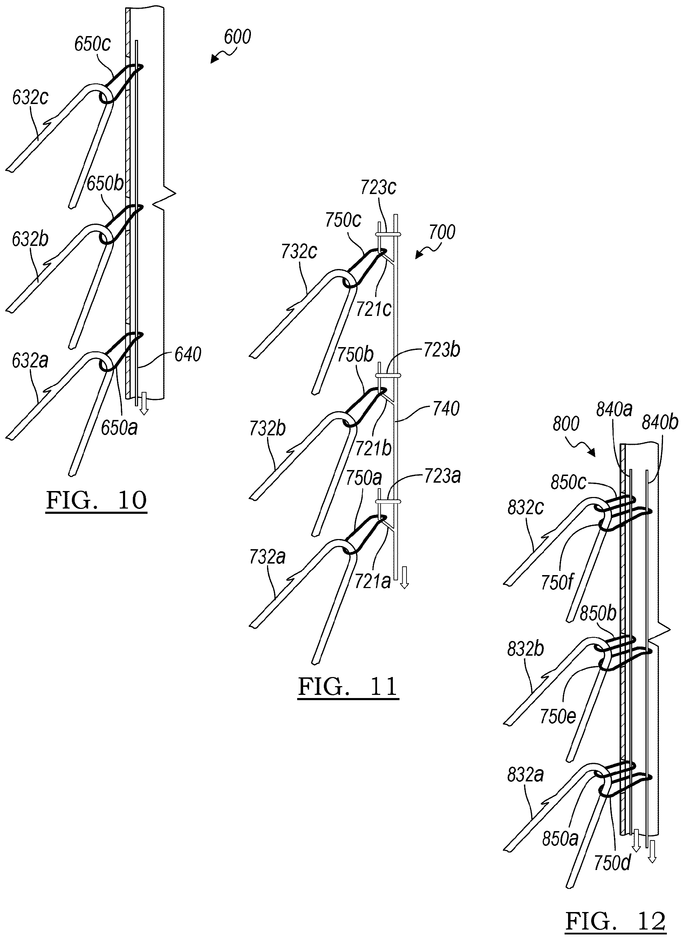

A delivery system constructed in accordance with the principles of the present disclosure may provide a number of structural features that allow for varying release schemes. In system 600 shown in FIG. 10, the loops 656a/656b/656c are each connected to a respective apex 634a/634b/634b of top stent 632, with a single trigger wire 640 passing through all loops 656a/656b/656c, to define an attached or delivery state. When the trigger wire 640 is pulled distally, loop 650c will release first, followed by loop 650b, then loop 650a. This represents a sequential or peak-by-peak release scheme into the detached, or delivered, state.

The delivery system 700 of FIG. 11, by contrast, allows for simultaneous release of the apices 734a/734b/734c. It does so by the construction of the trigger wire 740, which includes branches 721a/721b/721c angled away from the main body of the trigger wire 740. The loops 756a/756b/756c are held in place by respective sleeves or restraints 723a/723b/723c. When trigger wire 740 is pulled distally, the loops 756a/756b/756c will slide an equivalent distance along branches 721a/721b/721c until they encounter and dislodge restraints 723a/723b/723c and release.

In another example, delivery system 800, shown in FIG. 12, provides for a sequential stepwise release of the apices 834a/834b/834c. In this system 800, two trigger wires 840a and 840b are employed, with two sets of loops 850/852 associated with one or both trigger wires 840a/840b. As illustrated in FIG. 12, trigger wire 840a passes through small loops 856a/856b/856c, and both trigger wires 840a and 840b pass through large loops 852a/852b/852c. In a variation on this construction, the loops 852a/852b/852c are only operably connected to the second trigger wire 840b.

In one release scheme, trigger wire 840a may first be pulled distally to allow the top stent 832 a greater degree of movement and/or expansion in the radial dimension. This may effectively cause a partial expansion of the stent graft 830, so that the final position of the stent graft 830 can be approximated in the vasculature of the patient, and repositioned by the practitioner as necessary. The second trigger wire 840b can then be pulled distally to fully release the stent graft 830 such that it fully expands in the body vessel of the patient. Such a stepwise release may reduce the chances that a barb prematurely engages the vessel wall.

Features of any of the systems 600/700/800 may be used in combination with any of the other systems 600/700/800 according to the intended application.

Although the present disclosure has been described with reference to examples and the accompanying drawings, the present disclosure is not limited thereto, but may be variously modified and altered by those skilled in the art to which the present disclosure pertains without departing from the spirit and scope of the present disclosure.

All optional and preferred features and modifications of the described embodiments and dependent claims are usable in all aspects of the invention taught herein. Furthermore, the individual features of the dependent claims, as well as all optional and preferred features and modifications of the described embodiments are combinable and interchangeable with one another.

The disclosures in United States patent application no. 62/939,219, from which this application claims priority, and in the abstract accompanying this application are incorporated herein by reference.

## Claims

1. A system for deploying at least a portion of a stent, the system comprising:
a cannula comprising a lumen;
a guide member coupled to the cannula, the guide member comprising an outer surface, the guide member defining at least one curved channel therein, the at least one curved channel defining a trigger wire lumen, the guide member comprising at least one access port through the outer surface to the curved channel; and
a trigger wire disposed in the trigger wire lumen.

2. The system of claim 1, wherein the curved channel is a helical channel.

3. The system of claim 1 or 2, wherein the guide member defines two curved channels therein.

4. The system of claim 3, wherein the two curved channels do not intersect.

5. The system of any preceding claim, further comprising a sleeve disposed about at least a portion of the cannula.

6. The system of claim 5, wherein the sleeve is disposed about a portion of the trigger wire.

7. The system of any preceding claim, wherein the guide member comprises a plurality of access ports through the outer surface and in communication with the curved channel.

8. The system of any preceding claim, further comprising a tip disposed proximal the guide member, the tip defining a cavity in which a portion of the trigger wire is disposed.

9. The system of any preceding claim, further comprising a loop disposed about the trigger wire and extending at least partially into the access port.

10. The system of claim 9, comprising a first loop and a second loop, the first loop having a first length, the second loop having a second length greater than the first length.

11. The system of any preceding claim, further comprising a handle distal of the cannula for controlling the trigger wire.

12. The system of any preceding claim, wherein the access port comprises a smoothed or chamfered edge.

13. The system of any preceding claim, wherein the guide member has a substantially ovoid shape.

14. The system of any preceding claim, wherein the guide member comprises a helical tube.

15. A medical device assembly comprising:
a cannula comprising a lumen;
a guide member disposed over the cannula, the guide member comprising an outer surface, the guide member comprising at least one curved channel therein, the at least one curved channel defining a trigger wire lumen, the guide member comprising at least one access port through the outer surface to the curved channel;
a trigger wire disposed in the trigger wire lumen;
at least one loop disposed about the trigger wire and extending through the access port; and
a stent graft disposed about the cannula, the stent graft comprising an end stent defining an apex, the apex extending through the loop.
